# EUROPEAN PATENT SPECIFICATION

(11) **EP 1 499 755 B1**
(45) Date of publication and mention of the grant of the patent: **21.05.2008**
(21) Application number: 03747498.8
(22) Date of filing: 23.04.2003
(51) Int. Cl.: C23C 8/20, C23C 8/36, C22C 29/02, C22C 19/07, A61F 2/30

(54) **SURFACE TREATMENT OF CO-CR BASED ALLOYS USING PLASMA CARBURIZATION**
OBERFLÄCHENBEHANDLUNG VON LEGIERUNGEN AUF CO-CR-BASIS UNTER VERWENDUNG VON PLASMAKARBURIERUNG
TRAITEMENT DE SURFACE D'ALLIAGES A BASE DE CO-CR PAR CEMENTATION AU PLASMA

(30) Priority: 29.04.2002 GB 0209797
(43) Date of publication of application: 26.01.2005
(73) Proprietor: AD.Surf.Eng., Birmingham B15 2SQ (GB)
(72) Inventor: BELL, Thomas, Blundellsands, Merseyside L23 6SX (GB); DONG, Hanshan, Birmingham B29 6QP (GB); LI, Chenxi, Birmingham B29 7QP (GB)
(74) Representative: Ward, David Ian
(86) International application number: PCT/GB2003/001702
(87) International publication number: WO 2003/093527

(56) References cited:
- EP-A- 0 801 142
- US-A- 4 193 825
- US-A- 5 308 412
- US-A- 5 380 547
- US-A- 5 498 302

## Description

The present invention relates to a process for producing a superior wear-resistant surface on cobalt-chromium (Co-Cr) based medical implants. Particularly but not exclusively, it relates to a surface hardening process applicable to cobalt-chromium (Co-Cr) based metal-on-metal orthopaedic implant devices (prostheses), wherein surface hardness and wear resistant properties of the prostheses are enhanced without loss of corrosion resistance.

Cobalt-chromium based alloys have typically been used for orthopaedic applications because of their strength, resistance to wear and corrosion and biocompatibility. However, under conditions of sliding wear or articulation of the Co-Cr alloy against other bearing surfaces (particularly, ceramics or Co-Cr alloy counterfaces), the cobalt-chromium alloy produces wear debris from the counterface surfaces in relative motion. This raises a major concern over the carcinogenic effect of such Co-Cr wear debris and the release of such metal ions as Co and Cr. Therefore, the surface of the Co-Cr alloys must be hardened in order to minimise wear, thus leading to long-life Co-Cr orthopaedic prostheses. Several methods for improving wear resistance of Co-Cr based alloys and such articles made from these alloys have been attempted.

One approach to enhancing the wear performance of metallic femoral components (hip and knee prostheses) is to coat their surfaces with such ceramic coatings as TiN (J.A. Davidson, Ceramics in substitutive and reconstructive surgery (P. Vincenzini ed), Elsevier, Amsterdam, 1991 pp157-166) and more recently diamond-like carbon (DLC) coating (M. Allen, J. Biomed. Mater. Res., 58(2001), pp319-328). Potential drawbacks to this approach are concerns over spallation of coatings (M.T. Raimondi and R. Pietrabissa, Biomaterials, 21 (2000), pp907-913) due to the non-metallurgical bonding and galvanic corrosion (caused by the large galvanic potential difference and pinholes in coatings) (R.S. Lillard et. al., Surface Engineering 15 (1999), pp221-224). Although a TiN coating has been explored for use as a bearing surface against UHMWPE since the late 1980's, laboratory testing and clinical results have been limited. Indeed, it was reported in 1998 that 5,000 hip operations might have to be repeated in the UK, the problem being attributed to the TiN-coated surface (R. Ellis et. al., The Times, 19 February 1998, p1.). Although simple configuration laboratory tests indicated that the DLC coatings had exceptional friction and wear characteristics, tests on hip simulators produced early failures of the coatings (A.H.S. Jones and D. Teer 'Friction and wear testing of DLC type coatings on total hip replacement prostheses' Seminar on The Friction Lubrication and Wear of Artificial Joints - Tribology Meets Medical, Institute of Mechanical Engineers, 30 November 2000, Leeds). Clearly there is cause for concern in using coatings for metallic femoral components. The problem is mainly due to the formation of an oxide scale (Cr₂O₃) on the alloy surface due to the strong affinity of chromium, which is a major alloying element of Cr-Co alloys, with oxygen in air. This oxide scale frequently cause a poor adhesion between a coating and the Co-Cr alloy surface. Surface treatment of Co-Cr alloys usually has to overcome this major problem, and consequently, such coating techniques as PVD coating, electroplating, and electrolysis plating have limitations for Co-Cr alloys, as compared with coating and plating for most ferrous alloys.

Another approach to improving the wear performance of femoral components is to modify the metallic surface. In this respect, ion implantation with nitrogen has been employed since the mid 1980's to improve wear resistance of metallic bearing surfaces made of Ti-6AI-4V, 316L and Co-Cr-Mo 0.1. Onate, Surface and Coatings Technology, 142-144 (2001), pp1056-1062). However, it should be noted that the effectiveness of ion implantation in enhancing wear resistance of Co-Cr-Mo alloys for metal-on-metal articulation is limited by the inherent line-of-sight nature of the ion beam and the very thin modified layer which is produced. It is difficult, if not impossible, to produce a homogeneous surface modified layer on 3-D complexly shaped prostheses using the line-of-sight ion beams. In addition, the thickness of the modified surface layer (normally in the range of 0.01 to 0.2 µm) is far less than the average annual linear wear rate of Co-Cr-Mo prostheses.

In addition to ion implantation with nitrogen, known surface-hardening methods include gas nitriding or plasma nitriding. A nitriding process is disclosed in detail in US Patent No. 5,912,323 issued on May 3 1994 entitled "METHOD OF SURFACE HARDENING COBALT-CHROMIUM ALLOYS FOR ORTHOPAEDIC IMPLANT DEVICES", which disclosure is hereby incorporated by reference. To produce a measurable hardened layer, a treatment duration as long as 48 hours needs to be used. The thickness of the effectively hardened layer is very small as evidenced by the statement that "the peak nitrogen concentration occurs at a depth between 10 and 100nm". This is largely due to the strong affinity between nitrogen and chromium. Indeed, it was found that after plasma nitriding at 550°C for 8h in a 75%N₂-25%H₂ gas mixture, only a compound layer of CrN was observed in the nitrided case of Stellite 6B, a Co-30Cr alloy without the formation of an appreciable diffusion zone (P.H. Howill, Ion nitriding stellite in T. Spaluins and W.L. Kovaes (eds): Proceedings of 2nd International Conference of Ion Nitriding/Carburising. ASM International 1990, 175-176).

US 5,498,302 discloses a method of producing surface hardened, abrasion resistant, high strength medical implants. The loss of corrosion resistance which normally accompanies standard precipitation hardening is overcome by the use of a small quantity of a metal solute which is more nitridable or oxidizable than the other elements of the alloy.

EP 0 801 142 concerns a procedure fur the surface treatment of a metal species, and more specifically, a surface hardening procedure that works by enriching a superficial surface layer of a sample with carbon or boron. The treatment is said to be suitable for metal alloys having fcc, bcc or tetragonal structure, more particularity to components made from austenitic or martensitic stainless steel, nickel- or cobalt-based alloys. The procedure can also be applied to components fabricated from aluminum or titanium-based alloys. Examples of components include targets, valves, seats and covers, nuts and bolts, couplings, transport rollers, many parts in pumps, knives, orthopaedic implants, vanes, turbine components and condensers.

It is an object of the present invention to provide an improved method of treatment of Co-Cr based alloy medical implants, which can enable the above-mentioned disadvantages to be obviated or mitigated.

In general, the present invention provides an improved surface hardening process which is relatively cost-effective and which is capable of producing, at a relatively low temperature, combined improvement in wear and corrosion resistance of Co-Cr based alloy joint prostheses (such as hip and knee joints).

According to the present invention, there is provided a method of modifying a surface characteristic of a cobalt-chromium based alloy medical inplant in accordance with claim 1.

The surface characteristic to be modified by the method of the present invention may be any one or more of hardness, wear resistance, corrosion resistance and fatigue strength.

Preferably, said medical implant is a joint or knee prosthesis. The plasma treating is preferably carried out at a temperature in the range of from 350 to 550°C, and more preferably 400 to 500°C. At these temperatures, the method generally increases wear resistance and corrosion resistance.

Preferably, said treatment pressure is in the range of from 400 to 600 Pa and is more preferably about 500 Pa.

Preferably, the duration of said treatment is in the range of from 2 to 50 hours and more preferably 5 to 30 hours.

Preferably, the or each carbon-containing gas is selected from a hydrocarbon (eg. methane), carbon dioxide and carbon monoxide.

Preferably, the plasma treatment is carried out in the presence of at least one unreactive gas, for example selected from hydrogen, helium, argon or other noble gas. As used herein "unreactive" relates to a gas which does not become incorporated into the article to any significant extent.

Preferably, the plasma treatment is carried out in the presence of at least one reactive gas, such as a nitrogen containing gas (eg. N₂ or ammonia). As used herein "reactive" relates to a gas which (or a part of which) does become incorporated into the article to a certain extent. Where a nitrogen-containing gas is used, the plasma treating step is preferably effected at a temperature of from 300 to 500°C.

Particularly preferred gas mixtures are hydrogen and methane, and hydrogen, argon and methane.

Preferably, the or each carbon-containing gas constitutes from 0.5 to 20% by volume of the total atmosphere. Preferably, said reactive gas (when present) constitutes from 0.5 to 10% by volume of the total atmosphere.

Preferably, said plasma treatment is effected in the absence of oxygen.

The method may include an article cleaning step prior to the plasma treatment step to remove any oxide scale. Preferably, said cleaning is effected by sputter cleaning (i.e. bombardment of the article surface with positive ions). Said cleaning step may be effected at or below the plasma treatment temperature in an atmosphere of one or more of gases selected from hydrogen, helium, argon or other noble gas.

It will be understood that after plasma treating, the article will be cooled. The rate of cooling may be anything from 0.1°C/min up to 1000°C/min. Cooling may be achieved by slow cooling in the plasma treating atmosphere or by fast cooling by quenching in a liquid. In order to prevent dimension distortion and oxidation, slow cooling in the plasma treating atmosphere is preferred.

A passivation and/or polishing step may be desirable after completion of the plasma treatment.

The present invention also resides in a surface-hardened cobalt-chromium based medical implant producible by the method of the present invention, said medical implant characterised by having:-
(i) a surface region comprising a supersaturated solid solution of carbon in cobalt.

Preferably, said surface region has a thickness in the range of from 3 to 50 µm.

The nature of the Co-Cr based alloy is not particularly limited, and for example, any other alloying ingredients such as molybdenum, nickel, tungsten and titanium may be included in the alloy composition. Carbon may also be included, preferably in the range of from 0.04 to 1.6wt%, more preferably in the range of 0.04 to 0.4wt% in the case of a medical implant, and 0.4 to 1.6wt% in the case of an engineering component.

Among the Co-Cr based alloys which are useful for joint prostheses are ASTM F75 (ISO5832/4), ASTM F799 (ISO5832/12), ASTM F90 (ISO5832/5) and ASTM F562 (ISO5832/6) or their equivalents with different trade names. Articles formed of these alloys which can be surface treated at a relatively low temperature (300-500°C) in accordance with the present invention include conventional hip and knee joint prostheses, metal-on-metal advanced bone conservation prostheses, dental implants and other implant devices. Among the Co-Cr based alloys which are useful for wear and/or corrosion resistant engineering components are Stellite 6B, Stellite 6K, MP35N and Ultimet. Wear-resistant engineering components made of these Co-Cr based alloys which can be surface treated at a relatively high temperature (600-700°C) include knifes and blades for chemical and food processing industrials, valves and pumps in the chemical and power industrials, bushings and steel mill equipment although such articles are not within the scope of the present invention. Among the Co-Cr based alloys which are useful for hardfacing deposits are the Stellite family (more than 20 alloys) and ERCoCr alloys (ERCoCr-A, -B, -C or -E). Articles deposited with these hardfacing alloys which are preferably surface treated at a relatively high temperature (300-700°C) include valves, dies, punches, moulds, turbine blades and knifes although such articles are not within the scope of the present invention.

Embodiments of the present invention will now be described by way of example only, with reference to the accompanying drawings in which:-
Fig 1 is a schematic view of a dc plasma unit in which the treatment described in the preferred embodiments below was effected,
Fig 2 is a micrograph of the cross-sectional microstructure of a Co-Cr-Mo test piece treated in accordance with the present invention,
Fig 3 shows XRD patterns for untreated test pieces and test pieces surface hardened in accordance with the present invention, and
Figs 4-6 are graphs showing the properties of untreated test pieces and test pieces surface hardened in accordance with the present invention.

Typical examples of suitable Co-Cr based alloys which are susceptible to the process of the present invention are summarised in Table 1. The Co-Cr based alloys of which the article is formed may be in the wrought, cast, hardfacing deposit or PM/HIP form before the article is subjected to the process to the present invention.

**TABLE 1: Examples of useful Co-Cr based alloys**

| **Alloy** | **Cr** | **Mo** | **Ni** | **W** | **C** | **Ti** | **Co** |
|---|---|---|---|---|---|---|---|
| *Wear-corrosion resistant biomedical alloys* | | | | | | | |
| F75 (ISO5832/4) | 27-30 | 5-7 | < 1 | - | <0.35 | - | bal |
| F799 (ISO5832/12) | 26-30 | 5-7 | < 1 | - | < 0.35 | - | bal |
| F90 (ISO5832/5) | 19-21 | - | 9-11 | 14-16 | <0.15 | - | bal |
| F562 (ISO5832/6) | 19-21 | 9-11 | 33-37 | - | <0.15 | <1 | bal |

| *Wear-resistant alloys* | | | | | | | |
|---|---|---|---|---|---|---|---|
| Alloy 6B | 28-32 | <1.5 | <2.5 | 3-5 | 0.8-1.2 | - | bal |
| Alloy 6K | 28-32 | <1.5 | < 2.5 | 3.5-5.5 | 1.5-1.7 | - | bal |

| *Corrosion-resistant alloys* | | | | | | | |
|---|---|---|---|---|---|---|---|
| MP35N | 19-21 | 9-11 | 34-36 | - | - | - | bal |
| Ultimet | 25-27 | 4-6 | 8-10 | 1-3 | <0.06 | - | bal |

| *Hardfacing alloys* | | | | | | | |
|---|---|---|---|---|---|---|---|
| ERCoCr-A | ~28 | - | - | ~5 | ~1.2 | - | bal |
| ERCoCr-B | ~29 | - | - | ~8 | ~1.5 | - | bal |
| ERCoCr-C | ~31 | - | - | ~13 | ~2.5 | - | bal |
| ERCoCr-E | ~27 | ~6 | - | ~8 | ~0.2 | - | bal |

The surface-treatment-process can be applied as a final procedure without causing deterioration of the properties of the substrate or dimensional distortion of the article. There is no particular limit in the size of articles that can be treated using the process of the present invention.

In order to demonstrate the advantages of the present invention, a series of Co-Cr based alloys (Table 2) were treated in accordance with the present invention.

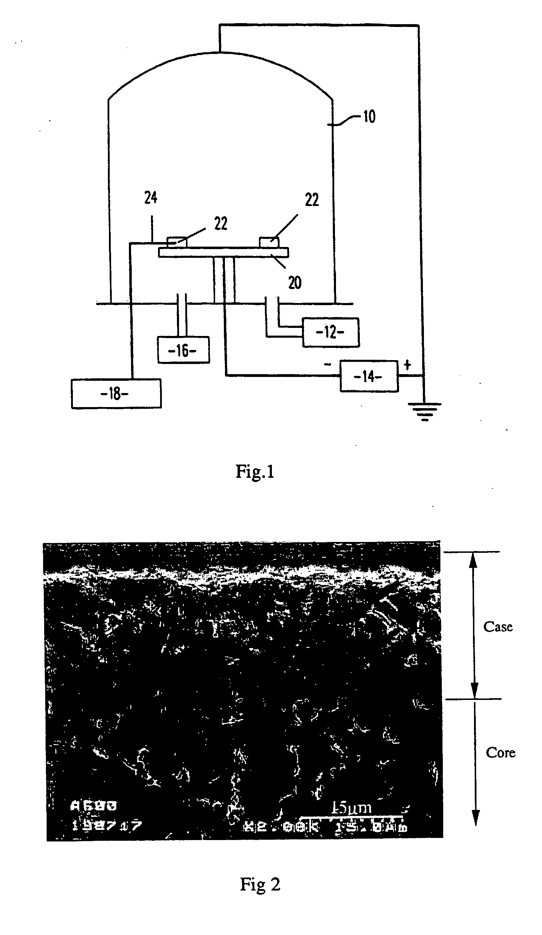

In the Examples, surface treatment was carried out using a dc plasma nitriding apparatus shown in figure 1. The apparatus comprises a sealable vessel 10, a vacuum system 12 with a rotary pump (not shown), a dc power supply and control unit 14, a gas supply system 16, a temperature measurement and control system 18 including a thermocouple 24, and a work table 20 for supporting articles 22 to be treated.

**TABLE 2: Alloy composition of the Examples**

| **Sample designation*** | **Condition** | **Composition (% by wt)** |
|---|---|---|
| A | Wrought | Co-27.6Cr-5.5Mo-0.06C |
| B | Wrought | Co-37.4Cr-6.1Mo-0.19C |
| C | Cast (MMT) | Co-29.2Cr-6.1Mo-0.21C |
| D | Cast (DEP) | Co-27.2Cr-0.17C |
| E | Cast (DRILL) | Co-29.6Cr-5.9Mo-0.05C |
| F | PM/HIP | Co-28.2Cr-5.8Mo-0.04C |

| | | |
|---|---|---|
| *For each sample designation, a different sample was treated at 400, 500 and 600°C (designated A400, A500, A600 etc.) | | |

The articles 22 to be treated were Co-Cr based alloy discs 25mm in diameter and 8 mm in thickness. The discs were placed on the table 20 inside the vessel 10. The table 20 was connected as a cathode to the power supply and control unit 14, and the wall of the vessel 10 was connected to the dc source as the anode. The temperature of the discs 22 was measured by the thermocouple 24 inserted into a hole of 3 mm diameter drilled in one of the discs 22 or a dummy sample. After the sealable vessel 10 was tightly closed, the rotary pump was used to remove the residual air (oxygen) and thus reduce the pressure in the vessel. When the reduction in pressure reached 10 Pa (0.1 mbar) or less, a glow discharge was introduced between the article 22 (cathode) and the vessel wall (anode) by applying a voltage of 400 volts to 900 volts between these two electrodes. A heating gas of hydrogen was at the same time introduced into the vessel 10. The pressure of the hydrogen gas in the vessel 10 was increased gradually as the temperature of the articles 22 increased. No external or auxiliary heating was employed, and the articles 22 were heated by the glow discharge only.

In other embodiments (not shown), an external heater attached to the vessel may be employed, or a combination of external heating and electrical glow discharge heating may be employed. Direct current (dc) discharge, pulsed dc discharge or alternating current (ac) discharge may be used.

After the articles 22 were heated to the prescribed temperature, a gas mixture of hydrogen (98.5%) and methane (1.5%) was introduced into the vessel 10 and the plasma treatment started. Treatment temperatures from 400°C to 600°C were employed for a treatment time of 10 hours. The working pressure in the treatment step was 500 Pa (5.0 mbar) for all the Examples.

During the plasma heat treatment, the methane is ionised, activated and dissociated to produce carbon ions and activated carbon atoms and neutral molecules, which then diffuse into the surface of the disc forming a carbon diffusion layer. When the plasma treatment is carried out at a relatively low temperature ranging from 300 to 550°C, the carbon atoms mainly reside in the cobalt lattices, forming a supersaturated solid solution with a possible nanocrystalline structure due to the relatively low temperatures employed in the treatment. The resultant layer has a high hardness, good fatigue strength and excellent wear and corrosion resistance (see below). When the plasma treatment is carried out at a relatively high temperature ranging from 600 to 700°C, is outside the scope of the present invention the, carbon atoms partially reside in the cobalt lattices forming a supersaturated solid solution and partially combined with carbon forming chromium carbides. The resultant layer has a high hardness, fatigue strength and excellent wear resistance.

After the completion of the plasma treatment, the glow discharge was turned off and the articles 22 were allowed to cool in the vessel 10 in the treatment atmosphere down to room temperature before they were removed from the vessel.

The articles 22 were then subjected to X-ray diffraction analysis for phase identification, glow discharge spectrometry (GDS) analysis for chemical composition determination, surface hardness measurements, metallography analysis of the cross section, electrochemical corrosion tests and wear tests.

A typical micrograph showing the cross-section of the carburised sample is given in figure 2. The sample was electrolytically etched in a 10% H₂SO₄ water solution. It can be seen that the carburised specimen is characterised by an "un-etched layer" on the surface followed by a carbon diffusion layer ("case") beneath and the heavily etched substrate ("core"). The un-etched layer is dense, no details can be revealed even under high resolution FEG-SEM. The thickness of the total carburised case depth increases with increasing carburising temperature. In conjunction with the chemical composition analysis, the case depth was determined to be 3.1, 9.3 and 20.2 µm respectively for the A-sample treated at 400°C, 500°C and 600°C for 10 hours.

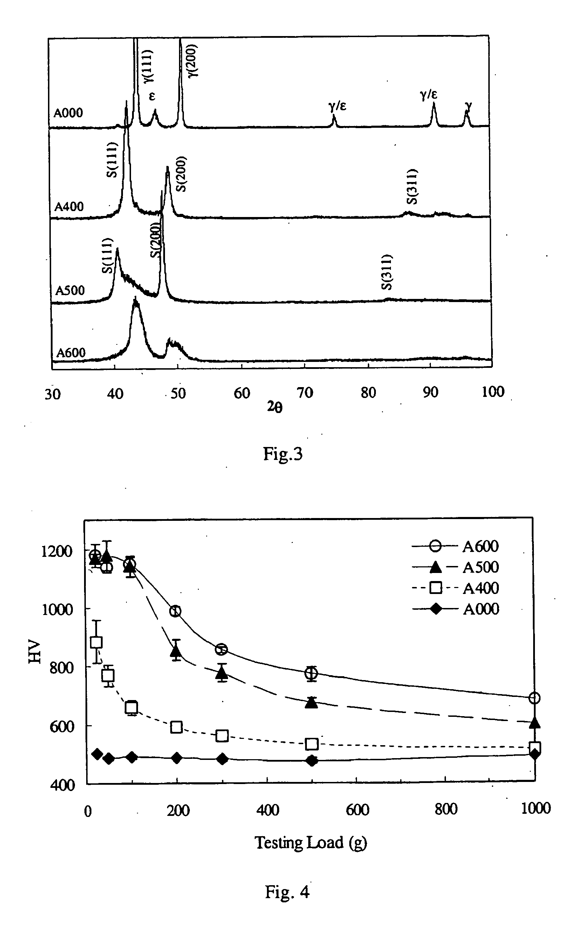

Figure 3 shows the XRD patterns for the untreated and carburised Co-27.6Cr-5.5Mo-0.06C alloy (Sample A). As can be seen, the untreated alloy consists of a mixture of f.c.c. structured γ-Co and h.c.p. structured ε-Co. It can be deduced from the height of the XRD peaks that the untreated alloy contains mainly γ with a small amount of ε. Plasma carburising has changed the phase constituent in the alloy surface. As shown in figure 3, only two main diffraction peaks at lower angles and some minor peaks at higher angles are detected. These peaks could not be matched to either γ-Co, ε-Co or any other phases given in the existing Powder Diffraction Database. However, they exhibit the same characteristics as those generated by the S-phase in plasma nitrided or carburised stainless steel (Y. Sun X. Li and T. Bell Surface Engineering, 1999, Vol 15, No. 1, pp49-54). It thus follows that S-phase was indeed produced in the plasma carburised Co-Cr-Mo alloy surface. The two main S-phase peaks, indicated as S(111) and S(200), correspond to the γ (111) and γ (200) of the substrate but are at lower angles. It is thought that the peak shift is caused by the solution of carbon which expands the f.c.c lattice structure of the substrate.

The surface mechanical properties of the plasma carburised samples were assessed using a Mitutoyo MVK-H tester under various loads, ranging from 0.025 kg to 1 kg. Figure 4 shows micro hardness measured on the sample A surface with various load. It can be seen that the hardness on the untreated surface (A000) is fairly stable under a testing load of above 0.05 kg, with an average value of HV 486. After carburising, the surface hardness of the Co-Cr-Mo alloy was increased under all testing loads. Under indentation loads below 0.1 kg, surface hardness values of more than 1100 HV were obtained for the 500°C and 600°C treated samples (A500 and A600 respectively). The surface hardness values for the 400°C treated sample (A400) were not as high as those for the 500°C and 600°C treated samples, but they are still much higher than those for the untreated alloy. As the testing load increased, all the measured hardness values decreased, showing a hardness gradient with testing load and this with the indentation depth. Such a diffusion type of hardness distribution is essential in ensuring optimum performance of surface treated system, since a sudden structural, compositional, and property change at the layer/core interface may lead to catastrophic interfacial failure of the layer during service. It was also found that the edges of the Vickers indentation impressions on the carburised samples were sharp and clear. No cracks were observed around or inside the indentation impression, and there was no evidence of interfacial failure between the carburised layer and the substrate even at a higher load of 1 kg. These observations suggest that the carburised layer possesses good ductility, high toughness and high load bearing capacity.

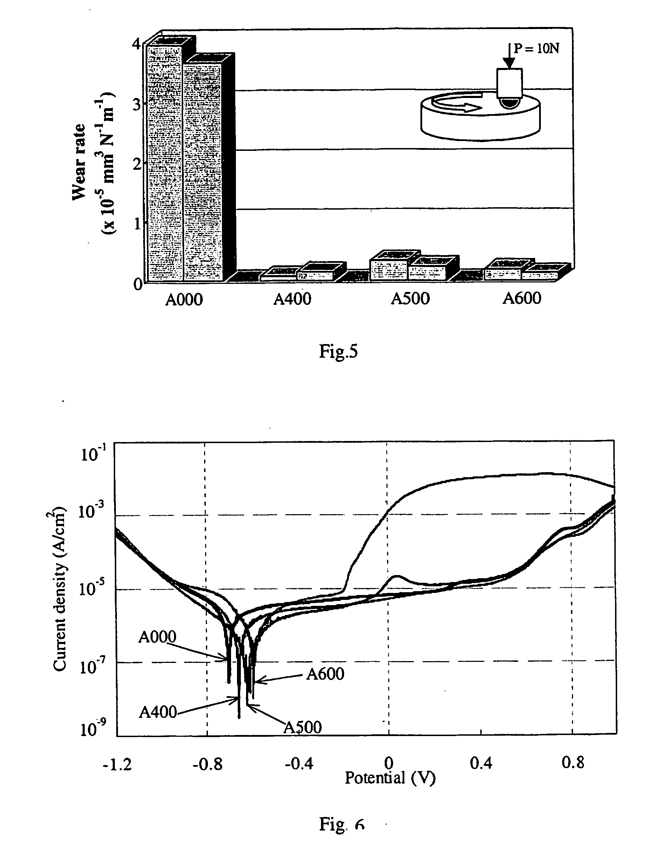

Plasma carburised and untreated test pieces were subjected to pin (WC ball)-on-disc wear tests sliding against 8 mm WC balls at a speed of 0.03 m/s with a initial maximum Hertzian contact stress of 1500MP. Wear rate, in terms of volume loss per meter sliding distance per Newton load (mm³m⁻¹N⁻¹), was calculated and is shown in figure 5. As can be seen from figure 5, the wear rate of all the carburised test pieces was dramatically reduced by more than one order of magnitude as compared with the untreated test pieces.

Electrochemical potentiondynamic sweep corrosion tests were conducted at room temperature in a flat cell with Ringer's solution which contained 9 g/l sodium chloride, 0.42 g/I potassium chloride, 0.48 g/I calcium chloride and 0.2 g/I sodium bicarbonate in distilled water. The polarisation curves are shown in figure 6. As compared with the untreated test piece, the corrosion potentials of all plasma carburised test pieces were moved to more passive values, indicating improved corrosion resistance. The current densities of both the untreated and the A400 and A500 test pieces are practically the same. The A600 test piece showed a higher current density scan potential was over -0.2V.

The applicability of the present invention is demonstrated in Table 3. Treatment was carried out at 500°C for 10 hrs in a gas mixture of methane and hydrogen. Microhardness was measured on the treated sample with a Vicker's indenter at a load of 0.1 kgf, indicated as HV0.1. As can be seen, all five types of Co-Cr based alloys in wrought, cast or PM/HIP form can be effectively surface hardened by applying the method of the present invention.

**TABLE 3: Surface hardness of samples A to F.**

| **Sample Number** | **Condition** | **Surface Hardness HV0.1** |
|---|---|---|
| A | Wrought | 1200 |
| B | Wrought | 1150 |
| C | Cast MMT) | 1200 |
| D | Cast (DEP) | 1000 |
| E | Cast (DRILL) | 1240 |
| F | PM/HIP | 960 |

In a modification of the above dc plasma method not shown), an advanced active screen plasma technology can be used to treat articles made of Co-Cr based alloys with improved surface quality. The articles to be plasma treated are placed inside a metal screen which is connected to the cathodic potential. The worktable and the articles to be treated are placed in a floating potential or subject to a relatively lower bias voltage (e.g. -100~-200V). As an example, a casting material (material D in Table 3) has been plasma treated in a active screen plasma unit and the surface hardness increased from <400HV0.1 (untreated material) to ~ 1050HV0.1 (active screen plasma treated).

## Claims

1. A method of improving surface hardness wear resistance or fatigue strength of a cobalt-chromium based alloy medical implant without loss in corrosion resistance, comprising plasma treating the implant at a temperature in the range of from 300 to 550°C and at a pressure of from 100 to 1500 Pa for 1 to 50 hours in an atmosphere comprising at least one carbon-containing gas, whereby to introduce carbon into a surface region of said implant, to produce a supersaturated solid solution of carbon in cobalt.

2. The method of claim 1 wherein the medical implant, is a joint or knee prosthesis.

3. The method of claim 1 or 2, wherein the plasma treating is carried out at a temperature in the range of from 350 to 550°C.

4. The method of claim 3, wherein the plasma treating is carried out at a temperature in the range of from 400 to 500°C.

5. The method of any preceding claim, wherein said treatment pressure is in the range of from 400 to 600 Pa.

6. The method of any preceding claim wherein the duration of said treatment is in the range of from 5 to 30 hours.

7. The method of any preceding claim, wherein the or each carbon-containing gas is selected from a hydrocarbon, carbon dioxide and carbon monoxide.

8. The method of any preceding claim, wherein the plasma treatment is carried out in the presence of at least one other gas selected from hydrogen, helium, argon or other noble gas.

9. The method of any preceding claim, wherein the plasma treatment is carried out in the presence of at least one additional gas to be incorporated into the surface region of said implant in addition to the carbon.

10. The method of claim 9, wherein the additional gas is a nitrogen containing gas.

11. The method of claim 9 or 10 wherein said additional gas constitutes from 0.5 to 10% by volume of the total atmosphere.

12. The method of claim 8, wherein the at least one other gas is hydrogen or a mixture of hydrogen and argon, and the carbon-containing gas is methane.

13. The method of any preceding claim, wherein the or each carbon-containing gas constitutes from 0.5 to 20% by volume of the total atmosphere.

14. The method of any preceding claim, wherein said plasma treatment is effected in the absence of oxygen.

15. The method of any preceding claim which includes a medical implant cleaning step prior to the plasma treatment to remove oxide scale.

16. The method of claim 15, wherein cleaning is effected by sputter cleaning.

17. The method of claim 15 or 16, wherein said cleaning step is effected at or below the subsequent plasma treatment temperature in an atmosphere of one or more gases selected from hydrogen, helium, argon or other noble gas.

18. The method of any preceding claim, wherein the medical implant is cooled after the plasma treatment.

19. The method of claim 18, wherein the rate of cooling is from 0.1°C/min up to 1000°C/min.

20. The method of claim 18 or 19, wherein the cooling is achieved by relatively slow cooling in the plasma treating atmosphere or by relatively fast cooling by quenching in a liquid.

21. The method of any preceding claim, wherein a passivation and/or polishing step is effected after completion of the plasma treatment.

22. The method of any preceding claim, wherein the Co-Cr based alloy includes one or more other alloying ingredients selected from molybdenum, nickel, tungsten, titanium and carbon.

23. A surface-hardened cobalt-chromium based alloy medical implant producible by the method of any one of claims 1 to 22, said implant **characterised by** having a surface region comprising a supersaturated solid solution of carbon in cobalt.

24. The medical implant of claim 23, wherein said surface region has a thickness in the range of from 3 to 50 µm.

25. The medical implant of claim 23 or 24 wherein the Co-Cr based alloy includes one or more other alloying ingredients selected from molybdenum, nickel, tungsten, titanium and carbon.

26. The medical implant of claim 25, wherein carbon is present as an alloying ingredient in an amount of from 0.04 to 1.6wt%.

27. The medical implant of claim 26, wherein carbon is present as an alloying ingredient in the range of from 0.04 to 0.4wt%.

28. The medical implant of any one of claims 23 to 27 which is a conventional hip or knee joint prostheses, a metal-on-metal advanced bone conservation prostheses, or a dental implant.

## Patentansprüche

1. Verfahren zur Verbesserung der Oberflächenhärte, Verschleißfestigkeit oder Dauerfestigkeit eines medizinischen Implantats aus einer Legierung auf Kobalt-Chrom-Basis ohne Verlust bei der Korrosionsresistenz, umfassend Plasmabehandlung des Implantats bei einer Temperatur im Bereich von 300 bis 550°C und bei einem Druck von 100 bis 1500 Pa für 1 bis 50 Stunden in einer Atmosphäre, welche mindestens ein kohlenstoffhaltiges Gas umfasst, um **dadurch** Kohlenstoff in einen Oberflächenbereich des Implantats einzuführen, um eine übersättigte feste Lösung von Kohlenstoff in Kobalt herzustellen.

2. Das Verfahren nach Anspruch 1, wobei das medizinische Implantat eine Gelenk- oder Knieprothese ist.

3. Das Verfahren nach Anspruch 1 oder 2, wobei die Plasmabehandlung ausgeführt wird bei einer Temperatur im Bereich von 350 bis 550°C.

4. Das Verfahren nach Anspruch 3, wobei die Plasmabehandlung ausgeführt wird bei einer Temperatur im Bereich von 400 bis 500°C.

5. Das Verfahren nach einem der vorangehenden Ansprüche, wobei der Behandlungsdruck im Bereich von 400 bis 600 Pa liegt.

6. Das Verfahren nach einem der vorangehenden Ansprüche, wobei die Dauer der Behandlung im Bereich von 5 bis zu 30 Stunden liegt.

7. Das Verfahren nach einem der vorangehenden Ansprüche, wobei das oder jedes kohlenstoffhaltige Gas ausgewählt wird aus Kohlenwasserstoff, Kohlenstoffdioxid und Kohlenstoffmonoxid.

8. Das Verfahren nach einem der vorangehenden Ansprüche, wobei die Plasmabehandlung ausgeführt wird in der Gegenwart von mindestens einem anderen Gas ausgewählt aus Wasserstoff, Helium, Argon oder einem anderen Edelgas.

9. Das Verfahren nach einem der vorangehenden Ansprüche, wobei die Plasmabehandlung ausgeführt wird in der Gegenwart von mindestens einem zusätzlichen Gas, um zusätzlich zu dem Kohlenstoff in den Oberflächenbereich des Implantates eingelagert zu werden.

10. Das Verfahren nach Anspruch 9, wobei das zusätzliche Gas ein stickstoffhaltiges Gas ist.

11. Das Verfahren nach Anspruch 9 oder 10, wobei das zusätzliche Gas 0,5 bis 10 Vol.-% der GesamtAtmosphäre ausmacht.

12. Das Verfahren nach Anspruch 8, wobei das zumindest eine andere Gas Wasserstoff oder eine Mischung von Wasserstoff und Argon ist, und das kohlenstoffhaltige Gas Methan ist.

13. Das Verfahren nach einem der vorangehenden Ansprüche, wobei das oder jedes kohlenstoffhaltige Gas von 0,5 bis 20 Vol.-% der Gesamtatmosphäre ausmacht.

14. Das Verfahren nach einem der vorangehenden Ansprüche, wobei die Plasmabehandlung bei Abwesenheit von Sauerstoff erfolgt.

15. Das Verfahren nach einem der vorangehenden Ansprüche, welches einen Reinigungsschritt des medizinischen Implantats vor der Plasmabehandlung einschließt, um Oxidablagerung zu entfernen.

16. Das Verfahren nach Anspruch 15, wobei die Reinigung durch Sputterreinigung erfolgt.

17. Das Verfahren nach Anspruch 15 oder 16, wobei der Reinigungsschritt ausgeführt wird bei oder unterhalb der nachfolgenden Plasmabehandlungstemperatur in einer Atmosphäre von einem oder mehreren Gasen ausgewählt aus Wasserstoff, Helium, Argon oder einem anderen Edelgas.

18. Das Verfahren nach einem der vorangehenden Ansprüche, wobei das medizinische Implantat nach der Plasmabehandlung gekühlt wird.

19. Das Verfahren nach Anspruch 18, wobei die Kühlrate von 0,1°C/Min bis zu 1000°C/Min beträgt.

20. Das Verfahren nach Anspruch 18 oder 19, wobei das Kühlen erreicht wird durch relativ langsames Kühlen in der Atmosphäre der Plasmabehandlung oder durch relativ schnelles Kühlen durch Abschrecken in einer Flüssigkeit.

21. Das Verfahren nach einem der vorangehenden Ansprüche, wobei ein Passivierungs- und/oder Polierschritt ausgeführt wird nach Vollendung der Plasmabehandlung.

22. Das Verfahren nach einem der vorangehenden Ansprüche, wobei die Legierung auf Co-Cr-Basis ein oder mehrere Legierungsbestandteile einschließt ausgewählt aus Molybdän, Nickel, Wolfram,Titan und Kohlenstoff.

23. Oberflächengehärtetes medizinisches Implantat aus Legierung auf Kobalt-Chrom-Basis, herstellbar durch das Verfahren nach einem der Ansprüche 1 bis 22, wobei das Implantat **dadurch gekennzeichnet ist, dass** es einen Oberflächenbereich hat, welcher eine übersättigte feste Lösung von Kohlenstoff in Kobalt umfasst.

24. Das medizinische Implantat nach Anspruch 23, wobei der Oberflächenbereich eine Dicke im Bereich von 3 bis 50 µm aufweist.

25. Das medizinische Implantat nach Anspruch 23 oder 24, wobei die Legierung auf Co-Cr-Basis ein oder mehrere Legierungsbestandteile einschließt ausgewählt aus Molybdän, Nickel, Wolfram,Titan und Kohlenstoff.

26. Das medizinische Implantat nach Anspruch 25, wobei Kohlenstoff als ein Legierungsbestandteil vorliegt in einer Menge von 0,04 bis 1,6 Gew.-%.

27. Das medizinische Implantat nach Anspruch 26, wobei Kohlenstoff als ein Legierungsbestandteil vorliegt im Bereich von 0,04 bis 0,4 Gew.-%.

28. Das medizinische Implantat nach einem der Ansprüche 23 bis 27, welches eine konventionelle Hüft- oder Kniegelenkprothese, eine weiterentwickelte knochenerhaltende Metall-Metall-Prothese oder ein Dentalimplantat ist.

## Revendications

1. Procédé d'amélioration de la dureté de surface, de la résistance à l'usure, ou de la résistance à la fatigue d'un implant médical en alliage à base de cobalt-chrome sans perte de résistance à la corrosion, comprenant un traitement au plasma de l'implant à une température dans la gamme de 300 à 550°C et à une pression de 100 à 1500 Pa pendant 1 à 50 heures dans une atmosphère comprenant au moins un gaz contenant du carbone, de façon à introduire du carbone dans une région de la surface dudit implant, pour produire une solution solide sursaturée de carbone dans le cobalt.

2. Procédé selon la revendication 1 dans lequel l'implant médical est une prothèse d'articulation ou de genou.

3. Procédé selon la revendication 1 ou 2, dans lequel le traitement au plasma est effectué à une température dans la gamme de 350 à 550°C.

4. Procédé selon la revendication 3, dans lequel le traitement au plasma est effectué à une température dans la gamme de 400 à 500°C.

5. Procédé selon l'une quelconque des revendications précédentes, dans lequel ladite pression du traitement est dans la gamme de 400 à 600 Pa.

6. Procédé selon l'une quelconque des revendications précédentes dans lequel la durée dudit traitement est dans la gamme de 5 à 30 heures.

7. Procédé selon l'une quelconque des revendications précédentes, dans lequel le ou chaque gaz contenant du carbone est choisi parmi un hydrocarbure, le dioxyde de carbone et le monoxyde de carbone.

8. Procédé selon l'une quelconque des revendications précédentes, dans lequel le traitement au plasma est effectué en présence d'au moins un autre gaz choisi parmi l'hydrogène, l'hélium, l'argon ou un autre gaz noble.

9. Procédé selon l'une quelconque des revendications précédentes, dans lequel le traitement au plasma est effectué en présence d'au moins un gaz supplémentaire devant être incorporé dans la région de la surface dudit implant en plus du carbone.

10. Procédé selon la revendication 9, dans lequel le gaz supplémentaire est un gaz contenant de l'azote.

11. Procédé selon la revendication 9 ou 10 dans lequel ledit gaz supplémentaire constitue 0,5 à 10% en volume de l'atmosphère totale.

12. Procédé selon la revendication 8, dans lequel ledit au moins un autre gaz est l'hydrogène ou un mélange d'hydrogène et d'argon, et le gaz contenant du carbone est le méthane.

13. Procédé selon l'une quelconque des revendications précédentes, dans lequel le ou chaque gaz contenant du carbone constitue 0,5 à 20% en volume de l'atmosphère totale.

14. Procédé selon l'une quelconque des revendications précédentes, dans lequel ledit traitement au plasma est effectué en l'absence d'oxygène.

15. Procédé selon l'une quelconque des revendications précédentes qui inclut une étape de nettoyage de l'implant médical avant le traitement au plasma pour enlever la pellicule d'oxyde de fer.

16. Procédé selon la revendication 15, dans lequel le nettoyage est effectué par nettoyage par pulvérisation.

17. Procédé selon la revendication 15 ou 16, dans lequel ladite étape de nettoyage est effectuée à la température du traitement au plasma ultérieur ou en dessous de ladite température, dans une atmosphère d'un ou plusieurs gaz choisis parmi l'hydrogène, l'hélium, l'argon ou un autre gaz noble.

18. Procédé selon l'une quelconque des revendications précédentes, dans lequel l'implant médical est refroidi après le traitement au plasma.

19. Procédé selon la revendication 18, dans lequel la vitesse de refroidissement va de 0,1°C/min jusqu'à 1000°C/min.

20. Procédé selon la revendication 18 ou 19, dans lequel le refroidissement est obtenu par un refroidissement relativement lent dans l'atmosphère du traitement au plasma ou par un refroidissement relativement rapide par trempage brusque dans un liquide.

21. Procédé selon l'une quelconque des revendications précédentes, dans lequel une étape de passivation et/ou de polissage est effectuée après l'achèvement du traitement au plasma.

22. Procédé selon l'une quelconque des revendications précédentes, dans lequel l'alliage à base de Co-Cr inclut un ou plusieurs autres ingrédients d'alliage choisis parmi le molybdène, le nickel, le tungstène, le titane et le carbone.

23. Implant médical en alliage à base de cobalt-chrome à surface durcie, susceptible d'être produit par le procédé selon l'une quelconque des revendications 1 à 22, ledit implant étant **caractérisé par** le fait d'avoir une région de la surface comprenant une solution solide sursaturée de carbone dans le cobalt.

24. Implant médical selon la revendication 23, dans lequel ladite région de surface a une épaisseur dans la gamme de 3 à 50 µm.

25. Implant médical selon la revendication 23 ou 24 dans lequel l'alliage à base de Co-Cr inclut un ou plusieurs autres ingrédients d'alliage choisis parmi le molybdène, le nickel, le tungstène, le titane et le carbone.

26. Implant médical selon la revendication 25, dans lequel le carbone est présent en tant qu'ingrédient d'alliage en une quantité de 0,04 à 1,6% en poids.

27. Implant médical selon la revendication 26, dans lequel le carbone est présent en tant qu'ingrédient d'alliage dans la gamme de 0,04 à 0,4% en poids.

28. Implant médical selon l'une quelconque des revendications 23 à 27 qui est une prothèse d'articulation de hanche ou de genou classique, une prothèse à conservation osseuse évoluée métal-sur-métal, ou un implant dentaire.
